Europäisches Patentamt

European Patent Office

Office européen des brevets

(11) Publication number: **0 536 197 B1**

(19)

## EUROPEAN PATENT SPECIFICATION

(45) Date of publication of patent specification: 26.07.95

(51) Int. Cl.[6]: **C07D 498/08**, A61K 31/495,
//(C07D498/08,307:00,267:00)

(21) Application number: 91911451.2

(22) Date of filing: 05.06.91

(86) International application number:
**PCT/EP91/01036**

(87) International publication number:
**WO 92/00302 (09.01.92 92/02)**

The file contains technical information submitted after the application was filed and not included in this specification

(54) PURE CRYSTALLINE FORM OF RIFAPENTINE.

(30) Priority: **29.06.90 EP 90112458**

(43) Date of publication of application:
**14.04.93 Bulletin 93/15**

(45) Publication of the grant of the patent:
**26.07.95 Bulletin 95/30**

(84) Designated Contracting States:
**AT BE CH DE DK ES FR GB GR IT LI LU NL SE**

(56) References cited:
**WO-A-90/00553**
**US-A- 4 002 752**

**CHEMICAL ABSTRACTS, vol. 112, no. 20, 14 May 1990 (Columbus, Ohio, US), R. LU et al.: "Crystal modification of rifandin", see page 410, abstract no. 185647p, & Kangshengsu 1989, 14(5), pages 301-306**

(73) Proprietor: **GRUPPO LEPETIT S.p.A.**
**Via Roberto Lepetit, 8**
**I-20020 Lainate (MI) (IT)**

(72) Inventor: **NEBULONI, Marino**
**Via C. Ferrari, 30**
**I-20017 Rho (MI) (IT)**
Inventor: **OCCELLI, Emilio**
**Via Castelnuovo, 18**
**I-20015 Parabiago (MI) (IT)**
Inventor: **CAVALLERI, Bruno**
**Via Pierlombardo, 25**
**I-20135 Milano (IT)**

(74) Representative: **Macchetta, Francesco et al**
**GRUPPO LEPETIT S.p.A.**
**Patent and Trademark Department**
**Via Roberto Lepetit, 34**
**I-21040 Gerenzano (Varese) (IT)**

EP 0 536 197 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

IL FARMACO EDIZIONE SCIENTIFICA, vol. 32, no. 7, July 1977, Societa Chimica Italiana (Rome, IT), G. PELIZZA et al.: "Polymorphism of rifampicin", pages 471-481, see page 471, summary; page 472, experimental

## Description

The present invention relates to the preparation of a new pure crystalline form of rifapentine, to the new pure crystalline form as such and to pharmaceutical formulations containing it.

This new pure crystalline form will be called pure Form I or pure polymorphic Form I throughout this description and claims.

Rifapentine is the international non-proprietary name of an antibiotic substance which was disclosed in US Patent 4,002,752 and was found to be particularly useful in the treatment of acute pulmonary infections, see for instance M. Tsukamura et al., Kekkaku (Tuberculosis, Japan), 1986, 61/12, (633-639); P.E. Varaldo et al., Antimicrobial Agents Chemother. (USA), 1985, 27/4, (615-618); Yi Lu et al., Chin. J. Antib. (China), 1987, 12/5, (341-344) and also L.B. Heifets et al, Am. Rev. Respir. Dis 1990; 141, 626-360 which describe the bactericidal activity of rifapentine against Mycobacterium avium.

The rifapentine product which is obtained according to the process of US 4,002,752 is generally a mixture of different solid forms of rifapentine base. These different forms show different stability, formulability and bioavailability characteristics. Generally they are solvates or mixtures of solvates (depending on the "crystallization" solvent or solvents). Also an amorphous form is obtained but no pure crystalline form of rifapentine base was described or produced by this process.

WO 90/00553 describes some hydrohalides of rifapentine in pure crystalline forms.

New and stable forms of rifapentine are still needed to meet the requirements of stability both under normal storage and formulation conditions in order to optimize the preparation, formulation, storage and delivery steps in an accurate, reliable and standardized way. Moreover, a well defined and stable crystalline habit of an active principle is oftentimes a precondition and even a guarantee of a reliable and accurate bioavailability pattern that is not subject to relevant batch to batch variations.

Sometimes also the excessive hygroscopicity of an active ingredient may represent a tricky problem in storage and formulation procedures. The crystalline form of rifapentine that we describe here is not found to be particularly hydroscopic under normal handling and storage conditions.

One object of this invention is therefore a hitherto undisclosed pure crystalline Form I of rifapentine, which is easily and reliably obtained in a pure form according to the process of the invention. With "pure crystalline Form I" it is intended that the described crystalline form is a solid constituted by crystals of rifapentine containing more than 95% of the whole rifapentine present in the sample in the crystalline Form I. Such crystalline pure form does not appear to have been never described or prepared.

This pure crystalline form proves to be stable and not hygroscopic under usual storage conditions and under usual formulation handlings. This stability throughout the formulation and storage process is of importance for a reliable bioavailability of the administered dose.

As mentioned above, another object of the invention is the process for preparing rifapentine pure crystalline Form I. The starting material of this process is a mixture of crystalline Form I and an ethanol solvate (called SII) which is in turn obtained from a crude or purified preparation of rifapentine which may be in any form including solvates and amorphous forms and their mixtures.

The starting Form I and SII mixture is transformed into pure crystalline Form I according to one of the following procedures:

a) contact with water or water-containing substances or prolonged exposure to a high degree of humidity
b) thermal treatment

Typically, the contact with water is obtained by suspending the mixture in water and then recovering pure crystalline Form I by filtration.

A typical example of water-containing substances is represented by the excipients of a wet granulation process. Also in this case the starting rifapentine material is entirely transformed into crystalline Form I but, of course, it will not be "pure" or easily purifiable but in admixture with the excipients used, which account normally for a large part of the final mixture.

Exposure to a high degree of humidity is typically obtained in a closed vessel or relatively closed vessel with a degree of relative humidity higher than 80% for at least about 24 hours. An exposure for several days to a humidity degree of about 70% does not produce any appreciable change in the starting mixture Form I + SII.

A typical thermal treatment capable of transforming a mixture of Form I and SII into pure crystalline Form I is represented by a treatment under reduced pressure (e.g. 0.2-0.4 mmHg, 26.6-53.3 Pa) at a temperature of 70-80°C until the transformation is complete. This transformation, under the above conditions, requires about 24-36 h.

In general a thermal treatment of a rifapentine mixture Form I + SII at a temperature between 50° and 130°C at a reduced pressure (in the range of about 0.2-0.4 mmHg) is sufficient to quantitatively transform it in pure Form I. The required time varies from a few days, typically 1 to 3 days, for the lower temperatures to a few minutes, typically 3-10 min, for the highest temperatures.

THE PHYSICAL CHARACTERISTICS OF RIFAPENTINE PURE CRYSTALLINE FORM I ARE THE FOLLOWING:

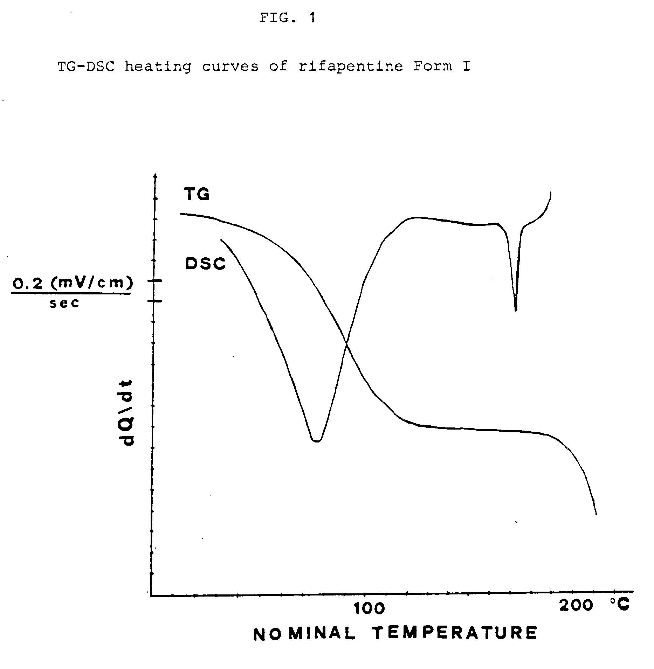

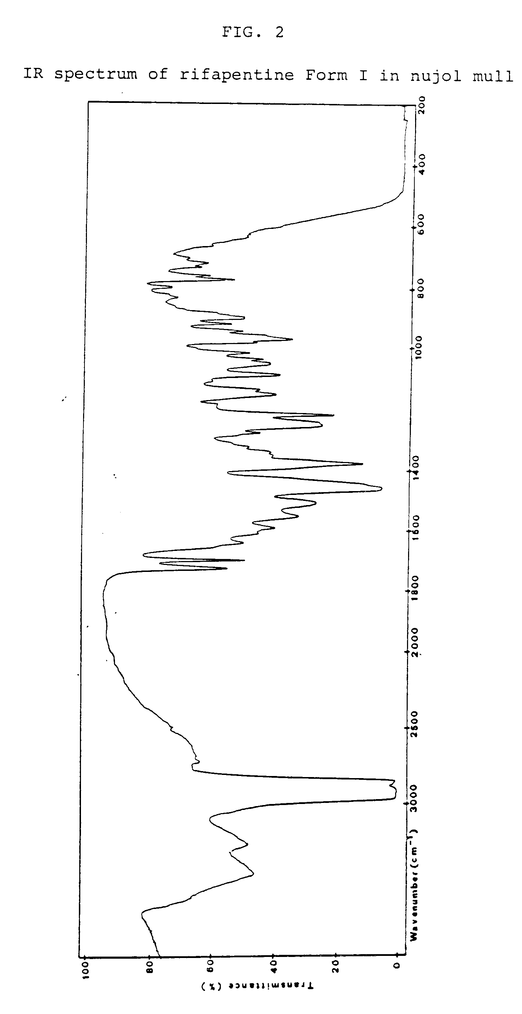

1. Solid state characterization
    Form I appears as anisotropic crystals (birefringence), with an undefined habit.
Crystalline habits and phase transitions are observed with a crystallographic microscope and a Kofler hot stage microscope (HSM).
2. Thermal analysis (DSC-TG) is conducted with a Mettler TA 2000 or a Du Pont 990 thermoanalyzer and with a thermobalance (Fig. 1) and the relative data are reported in Table I. The working conditions are: gas flow $N_2$ 25 ml/min, scan speed 10°C/min, weight of sample about 4 mg.
    Form I shows an endothermic peak at 172°C (due to the melting) immediately followed by the decomposition which prevents the heat of melting to be reliably determined. At 25-110°C a desolvation endotherm, due to imbibition water, occurs.
3. The Infrared spectrum (IR) is recorded with a Perkin Elmer mod. 580 spectrophotometer in nujol mull and is reported in Fig. 2, while the assignment of the main functional bands ($cm^{-1}$) is reported in TABLE II.

TABLE I

| M.P. or Transition temperature , °C | Weight loss, % |
|---|---|
| 25 - 110 * | 4 |
| 172 ** | - |

* Imbibition water
** Melting followed by rapid decomposition

TABLE II

| Ansa v OH | 3260 *,3440, 3280 |
|---|---|
| Furanone v C = O | 1730 |
| Acetyl v C = O | 1705 |
| Amide v C = O (amide I) | 1660, 1615 |
| Amide δ NH (amide II) | 1510 |
| Acetyl v C-O-C | 1250 |

* Narrow band of water

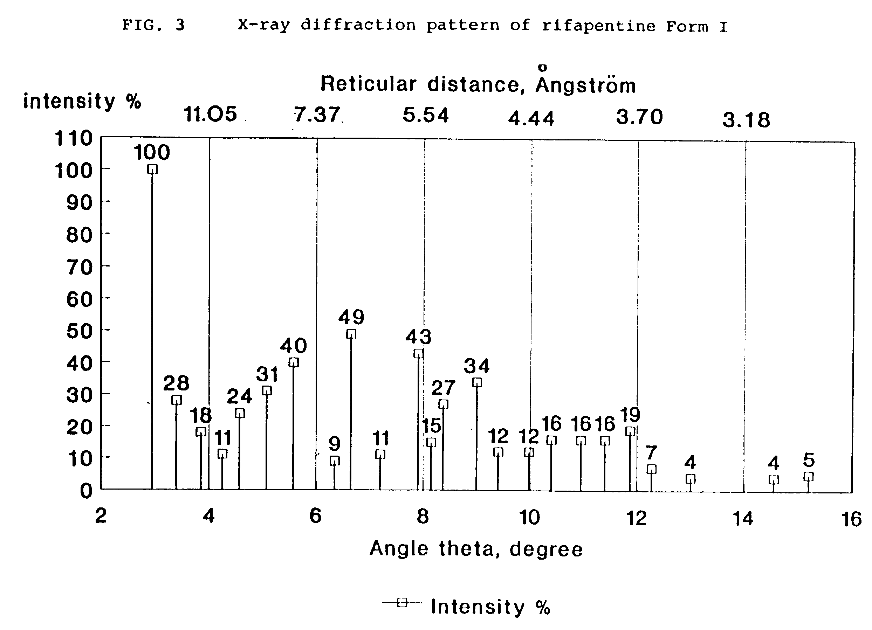

4. X-ray powder diffraction pattern of Form I according to ASTM, obtained with a Philips (PM 1010/77, 1049/01, 4025/10) instrument, radiation $CuK_{alpha}$, is reported in TABLE III and Fig. 3.

## TABLE III

Wawe-lenghts:   ALPHA 1         = 1.54051 Angström

               ALPHA 2         = 1.54433

               ALPHA - MEAN    = 1.54178

The five strongest reflections are:

| No. | Theta degree | D Angström | Intensity |
|-----|--------------|------------|-----------|
| 1   | 2.95         | 14.98      | 100       |
| 9   | 6.65         | 6.65       | 49        |
| 11  | 7.9          | 5.6        | 43        |
| 7   | 5.575        | 7.93       | 40        |
| 14  | 9            | 4.92       | 34        |

## TABLE III

Continued....

| No. | Theta degree | D Angström | Intensity |
|---|---|---|---|
| 1 | 2.95 | 14.98 | 100 |
| 2 | 3.4 | 13 | 28 |
| 3 | 3.85 | 11.48 | 18 |
| 4 | 4.25 | 10.4 | 11 |
| 5 | 4.575 | 9.66 | 24 |
| 6 | 5.075 | 8.71 | 31 |
| 7 | 5.575 | 7.93 | 40 |
| 8 | 6.35 | 6.97 | 9 |
| 9 | 6.65 | 6.65 | 49 |
| 10 | 7.2 | 6.15 | 11 |
| 11 | 7.9 | 5.6 | 43 |
| 12 | 8.15 | 5.43 | 15 |
| 13 | 8.375 | 5.29 | 27 |
| 14 | 9 | 4.92 | 34 |
| 15 | 9.4 | 4.72 | 12 |
| 16 | 9.975 | 4.45 | 12 |
| 17 | 10.4 | 4.27 | 16 |
| 18 | 10.95 | 4.05 | 16 |
| 19 | 11.4 | 3.9 | 16 |
| 20 | 11.875 | 3.74 | 19 |
| 21 | 12.275 | 3.62 | 7 |
| 22 | 13 | 3.42 | 4 |
| 23 | 14.55 | 3.06 | 4 |
| 24 | 15.2 | 2.94 | 5 |

As mentioned above, the starting material of the transformation process of the invention is represented by a mixture of rifapentine Form I and a solvate with ethanol (SII) which is conveniently referred to in this application as Form I + SII. This mixture is prepared by treating any forms of rifapentine, such as the solvates or mixtures obtained according to US 4,002,752, by the following procedure:

Rifapentine (any forms) is dissolved in a low boiling solvent, typically methylene chloride, after convenient stirring at room temperature, and preferably at about 30°C. Then ethanol is added to this solution and the volatile phase is partially removed by distilling at ambient pressure until a constant temperature of the mixture (about 75-80°C) is reached. Under these conditions all the low boiling solvent, (typically, methylene chloride) is in practice removed and the suspension that residuates contains the desired mixture rifapentine Form I + SII, which is recovered by filtration and then used as the starting material in the process of the invention.

Preferably, an equal volume of low boiling solvent and ethanol is used and, most preferably, the ratio between rifapentine (any forms) and ethanol is about 1 g per 25 ml. Ethanol is preferably 95% ethanol while the low boiling solvent is any solvent boiling between 20° and 70°C at ambient pressure which is capable of dissolving rifapentine at a temperature between room temperature and 30-35°C.

THE PHYSICAL CHARACTERISTICS OF RIFAPENTINE FORM I + SII ARE REPORTED BELOW:

1. Thermal analysis (DSC-TG) is conducted with a Mettler TA 2000 or a Du Pont 990 thermoanalyzer and with a thermobalance and the relative data are reported in Table IV. The working conditions are: gas flow $N_2$ 25 ml/min; scan speed 10°C/min, weight of sample about 4 mg.

TABLE IV

| Thermal parameters of rifapentine FORM I + SII | |
|---|---|
| M.P. or Transition temperature , °C | Weight loss, % |
| 30°-110°C * | about 3 |
| 140°-160°C ** | about 1.3 |
| 172°C *** | - |

\* = imbibition water
\*\* = desolvation of crystallization solvent [EtOH]
\*\*\* = melting followed by decomposition

2. Infrared spectrum (IR) is recorded with a Perkin Elmer mod. 580 spectrophotometer in nujol mull; the assignments of the main functional bands ($cm^{-1}$) are reported in TABLE V.

TABLE V

| Assignment of the main functional bands ($cm^{-1}$) of rifapentine FORM I + SII | |
|---|---|
| Ansa and ethanol v OH | 3620 *, 3440,3380, 3280, 3120 |
| Furanone v C = O | 1730 |
| Acetyl v C = O | 1710, 1700 |
| Amide v C = O (amide I) | 1680, 1660, 1615 |
| Amide δ NH (amide II) | 1500 |
| Acetyl v C-O-C | 1250, 1245 |

\* Narrow band of water

3. X-ray powder diffraction pattern of Form I + SII according to ASTM, obtained with a Philips (PW 1010/77, 1049/01, 4025/10) instrument, radiation $CuK_{alpha}$, is reported in TABLE VI.

## TABLE VI

Wawe-lenghts:  ALPHA 1        = 1.54051 Angström

ALPHA 2        = 1.54433

ALPHA – MEAN   = 1.54178

The five strongest reflections are:

| No. | Theta degree | D Angström | Intensity |
|-----|-----|-----|-----|
| 2 | 2.88 | 15.37 | 100 |
| 1 | 2.7 | 16.36 | 95 |
| 13 | 7.9 | 5.60 | 93 |
| 10 | 6.65 | 6.65 | 83 |
| 7 | 5.1 | 8.67 | 50 |

## TABLE VI

Continued....

| No. | Theta degree | D Angström | Intensity |
|---|---|---|---|
| 1 | 2.7 | 16.36 | 95 |
| 2 | 2.88 | 15.37 | 100 |
| 3 | 3.35 | 13.19 | 38 |
| 4 | 3.85 | 11.48 | 17 |
| 5 | 4.15 | 10.65 | 32 |
| 6 | 4.55 | 9.71 | 37 |
| 7 | 5.1 | 8.67 | 50 |
| 8 | 5.5 | 8.04 | 41 |
| 9 | 6.3 | 7.02 | 30 |
| 10 | 6.65 | 6.65 | 83 |
| 11 | 7.05 | 6.28 | 15 |
| 12 | 7.25 | 6.10 | 13 |
| 13 | 7.9 | 5.60 | 93 |
| 14 | 8.15 | 5.43 | 43 |
| 15 | 8.35 | 5.30 | 34 |
| 16 | 8.75 | 5.06 | 27 |

## TABLE VI

Continued....

| No. | Theta degree | D Angström | Intensity |
|---|---|---|---|
| 17 | 8.95 | 4.95 | 43 |
| 18 | 9.35 | 4.74 | 12 |
| 19 | 9.36 | 4.74 | 12 |
| 20 | 9.9 | 4.48 | 21 |
| 21 | 10.4 | 4.27 | 17 |
| 22 | 10.75 | 4.13 | 7 |
| 23 | 11.05 | 4.02 | 16 |
| 24 | 11.35 | 3.91 | 22 |
| 25 | 11.65 | 3.81 | 7 |
| 26 | 11.9 | 3.73 | 22 |
| 27 | 12.25 | 3.63 | 7 |
| 28 | 13 | 3.42 | 5 |
| 29 | 13.65 | 3.26 | 5 |
| 30 | 15.15 | 2.95 | 7 |
| 31 | 16.35 | 2.73 | 4 |

A further object of this invention is to provide pharmaceutical dosage forms containing rifapentine pure crystalline Form I. These pharmaceutical dosage forms include oral solid forms such as capsules, tablets, troches, film-coated tablets, sugar-coated tablets, hard gelatin capsules, soft elastic capsules, and the like. These forms can contain the pure crystalline rifapentine Form I in powder form admixed with the usual inert excipients such as binders, disintegrants, lubricants and preservatives. These additives are essentially the same which can be used in the formulations of similar antibiotics, e.g. rifampicin.

Other pharmaceuticals dosage forms which can be used for either oral administration or external applications include solutions, syrups, suspensions, emulsions and the like. Also in these cases the method and ingredients usually employed for the formulation of similar antibiotics, e.g. rifampicin, can be successfully utilized. Pharmaceutical dosage forms for parenteral administration are also comprised in this particular aspect of the invention. These pharmaceutical dosage forms include preparations suitable for intramuscolar or intravenous administration which optionally can be formulated as dry powder for reconstitution before use.

The rifapentine pure crystalline Form I may be also administered as medicated applications through the skin or various body orifices. Accordingly they are applied to the skin or inserted into body orifices in liquid, semi-solid or solid forms. This implies using pharmaceutical dosage forms like aerosol, ointments and suppositories.

The manufacture of the pharmaceutical dosage forms containing pure rifapentine Form I can be carried out by commonly known procedures, see for instance: Remington's Pharmaceuticals Sciences, 17 Edition, (1985) Mack Publishing Co., East, Pennsylvania 18042.

The above mentioned dosage forms show good long term stability under normal storage conditions.

EXAMPLE 1: Preparation of rifapentine polymorphic mixture Form I + SII (solvate with ethanol)

A suspension of rifapentine (2 g) in methylene chloride ($CH_2Cl_2$) (50 ml) is stirred at 30°C for 1 h until all the powder is solubilized. Then 50 ml of 95% ethanol is added and the solvents are removed by stripping at atmospheric pressure till a constant internal temperature is obtained (75-80°C).

The crystallization of rifapentine is observed at about 60°C. The final volume of the suspension is about 40-50 ml and the residual $CH_2Cl_2$ varies from 1 to 3% (gas-chromatographic determination) while the water content is in the range 2-5%.

Rifapentine is collected by filtration and washed with cold 95% ethanol.

The wet powder is dried in a static drier at 60°C under reduced pressure until a residual ethanol content less than 2% is reached (time required 6-8 hours).

The physical form of the obtained product is determined by microscopy, thermal analysis (DSC-TG), IR spectroscopy and X-ray powder diffraction. The relative data are reported in Tables IV, V and VI.

Evolved gas analysis confirmed that the solvent lost at 140-160°C was ethanol of crystallization.

EXAMPLE 2: Preparation of rifapentine pure crystalline Form I from a water suspension of rifapentine mixture Form I + SII

A suspension of rifapentine polymorphic mixture Form I + SII (1 g), obtained as described above, in about 100 ml of water is stirred for 4 hours at room temperature, than the solid is collected by filtration and washed three times with 10 ml of cold water. After drying under vacuum at 40°C, about 900 mg of rifapentine pure crystalline Form I is obtained as a powder showing small isotropic crystals with a needle habit which was analyzed by microscopy, thermal analysis (DSC-TG), IR spectroscopy and X-ray powder diffraction.

The relative data are reported in Tables I, II and III.

EXAMPLE 3: Preparation of rifapentine pure crystalline Form I by prolonged exposure of rifapentine polymorphic mixture Form I + SII to a high degree of humidity

A sample of rifapentine mixture Form I + SII is kept at room temperature at about 90% of relative humidity for 24 h. Under these conditions a complete transformation into rifapentine pure crystalline Form I is observed as shown by the physical analysis data which are in agreement with those reported above.

EXAMPLE 4: Preparation of rifapentine pure crystalline Form I by thermal treatment of rifapentine polymorphic mixture Form I + SII

When rifapentine mixture Form I + SII (particle size below 10 micrometers) is kept in an oven at a reduced pressure at the temperatures indicated below and for the reported time, its complete transformation into rifapentine pure crystalline Form I occurs.

| Pressure | Temperature (°C) | Time |
|---|---|---|
| 0.2-0.4 mmHg (26.6-53.3 Pa) | 50 | 2-3 days |
| | 70-80 | 36 h |
| | 100 | 6-10 h |
| | 130 | 5-10 min |

EXAMPLE 5: Physical stability of rifapentine pure crystalline Form I

a) Humidity

When rifapentine pure crystalline Form I is kept at room temperature at about 70% of relative humidity for 1 week the original state is maintained and no crystal modification occurs. The degree of crystallinity of Form I remains unchanged under these storage conditions.

a) Grinding

When a sample of rifapentine pure crystalline Form I is ground in a mortar or with a ballmill, the crystalline structure is broken and amorphous rifapentine is then recovered.

However, when Fitzmill or air-jet micronization is used, rifapentine pure crystalline Form I is recovered with a smaller particle size but without any significant change in its crystallinity.

EXAMPLE 6: Physical stability of rifapentine pure crystalline Form I during tablets formulation

Since physical processing can alter the crystallinity of a substance, a study was undertaken to monitor the crystallinity of rifapentine Form I in the different steps of the production of tablets.

Samples were taken during the granulation, drying, milling, blending, and tabletting process steps and their physical properties were evaluated by DSC-TG, IR spectroscopy and X-ray powder diffraction. The results confirm that rifapentine remains in Form I throughout all of the process steps and the degree of crystallinity is virtually the same for all samples.

Tablets containing rifapentine pure crystalline Form I are currently monitored for possible changes in the degree of crystallinity on long-term stability studies. The results shows that the pure crystalline Form I in these tablets is stable and there is no change in the degree of crystallinity after 1 year at 20°C or 35°C or at 35°C and 75% relative humidity. No chemical changes were detected under the same storage conditions.

## Claims

1. Rifapentine pure crystalline Form I having the following characteristics:
   a) Solid state characterization
      Form I appears as anisotropic crystals (birefringence), with an undefined habit;
   b) Thermal analysis (DSC-TG) is conducted with a Mettler TA 2000 or a Du Font 990 thermoanalyzer and with a thermobalance (Fig. 1) and the relative data are reported in Table I; The working conditions are: gas flow $N_2$ 25 ml/min, scan speed 10°C/min, weight of sample about 4 mg,
      Form I shows an endothermic peak at 172°C (due to the melting) immediately followed by decomposition which prevents the heat of melting to be reliably determined; at 25-110°C a desolvation endotherm, due to imbibition water, occurs;
   c) The Infrared spectrum (IR) is recorded with a Perkin Elmer mod. 580 spectrophotometer in nujol mull and is reported in Fig. 2, while the assignment of the main functional bands ($cm^{-1}$) is reported in TABLE II;

TABLE I

| M.P. or Transition temperature , °C | Weight loss, % |
|---|---|
| 25 - 110 * | 4 |
| 172 ** | - |

\* Imbibition water
\*\* Melting followed by rapid decomposition

TABLE II

| Ansa v OH | 3260 *,3440, 3280 |
|---|---|
| Furanone v C = O | 1730 |
| Acetyl v C = O | 1705 |
| Amide v C = O (amide I) | 1660, 1615 |
| Amide δ NH (amide II) | 1510 |
| Acetyl v C-O-C | 1250 |

\* Narrow band of water

d) X-ray powder diffraction pattern of Form I according to ASTM, obtained with a Philips (PM 1010/77, 1049/01, 4025/10) instrument, radiation CuK$_{alpha}$, is reported in TABLE III and Fig. 3

## TABLE III

Wawelenghts:  ALPHA 1    = 1.54051
                                  Angström

ALPHA 2    = 1.54433

ALPHA − MEAN  = 1.54178

The five strongest reflections are:

| No. | Theta degree | D Angström | Intensity |
|---|---|---|---|
| 1 | 2.95 | 14.98 | 100 |
| 9 | 6.65 | 6.65 | 49 |
| 11 | 7.9 | 5.6 | 43 |
| 7 | 5.575 | 7.93 | 40 |
| 14 | 9 | 4.92 | 34 |

2. A process for preparing the compound of claim 1 which comprises:
   a) contacting rifapentine Form I + SII with water or a water-containing substance or exposing to a relative humidity higher than 80% for at least 24 hours, or alternatively
   b) subjecting rifapentine Form I + SII to a thermal treatment at a temperature between 50° and 130°C

3. A process according to claim 2 wherein the contact with water is obtained by suspending rifapentine Form I + SII in water.

EP 0 536 197 B1

4. A process according to claim 3 wherein step (a) or alternative step (b) are conducted under reduced pressure.

5. A process according to claim 4 wherein the reduced pressure is in the range of 26.6 - 53.3 Pa.

6. A process according to claim 2, 4 or 5 wherein the thermal treatment is at a temperature between 70° and 80°C.

7. A process for preparing the compound of claim 1 which comprises:
   a) dissolving any forms of rifapentine in a low boiling solvent with stirring at room temperature, then
   b) adding ethanol and partially removing the volatiles by distilling at ambient pressure until a constant temperature of the residual mixture of about 75°-80°C is obtained,
   c) recovering the thus produced mixture of rifapentine Form I + SII by filtration, and
   d) contacting rifapentine Form I + SII with water or a water-containing substance or exposing it to a relative humidity higher than 80% for at least 24 hours under reduced pressure, or alternatively,
   e) subjecting it to a thermal treatment at a temperature between 50° and 130°C under reduced pressure.

8. A process according to claim 7 wherein the reduced pressure is in the range of 26.6 - 53.3 Pa.

9. A pharmaceutical formulation which contains rifapentine pure crystalline Form I as defined in claim 1 as an active ingredient.

10. A formulation according to claim 9 wherein the pharmaceutical formulation is an oral dosage form.

11. A formulation according to claim 10 wherein the oral dosage form is a tablet or a capsule.

12. A process for preparing a rifapentine pharmaceutical formulation which comprises admixing rifapentine pure crystalline Form I as defined in claim 1 with a pharmaceutically acceptable veichle.

13. A process according to claim 12 wherein the pharmaceutical formulation is in oral dosage form.

14. A process according to claim 13 wherein the oral dosage form is a tablet or a capsule.

**Patentansprüche**

1. Rifapentin der reinen kristallinen Form I mit folgenden Eigenschaften:
   a) Charakterisierung des festen Zustands
   Form I tritt als anisotrope Kristalle (Doppelbrechung) mit undefiniertem Kristallhabitus auf;
   b) Thermoanalyse (DSC-TG) wird mit einem Mettler TA 2000 oder Du Pont 990 Thermoanalysator und einer Thermowaage durchgeführt (Fig. 1) und die relativen Daten sind in Tabelle I aufgeführt; die Arbeitsbedingungen sind: Gasfluß $N_2$ 25 ml/Min., Scangeschwindigkeit 10°C/Min., Gewicht der Probe etwa 4 mg,
   Form I zeigt einen endothermen Peak bei 172°C (durch das Schmelzen), unmittelbar gefolgt von einer Zersetzung, die verhindert, daß die Schmelzwärme zuverlässig bestimmt werden kann; bei 25-110°C tritt eine Desolvatationsendothermie durch Imbibitions-Wasser auf;
   c) Das Infrarotspektrum (IR) wird mit einem Perkin Elmer Mod. 580 Spektrophotometer in Nujolsuspension aufgenommen und ist in Fig. 2 aufgeführt, während die Zuordnung der Hauptfunktionsbanden ($cm^{-1}$) in Tabelle II aufgeführt ist;

14

Tabelle I

| Schmp. oder Übergangstemperatur °C | Gewichtsverlust % |
|---|---|
| 25 - 110 * | 4 |
| 172 ** | - |

\* Imbibitions-Wasser
\*\* Schmelzen, gefolgt von schneller Zersetzung

Tabelle II

| Ansa v OH | 3260 *,3440, 3280 |
|---|---|
| Furanon v C = O | 1730 |
| Acetyl v C = O | 1705 |
| Amid v C = O (Amid I) | 1660, 1615 |
| Amid δ NH (Amid II) | 1510 |
| Acetyl v C-O-C | 1250 |

\* Schmale Wasserbande

d) Röntgen-Pulverbeugungsdiagramm der Form I gemäß ASTM, erhalten mit einem Philips (PM 1010/77, 1049/01, 4025/10) Instrument, Strahlung $CuK_{alpha}$, ist in Tabelle III und Fig. 3 aufgeführt.

## Tabelle III

Wellenlängen:   Alpha 1      = 1.54051 Angström

Alpha 2      = 1.54433

Alpha-Mittelwert  = 1.54178

Die fünf stärksten Reflexionen sind:

| Nr. | Theta Grad | D Angström | Intensität |
|---|---|---|---|
| 1 | 2.95 | 14.98 | 100 |
| 9 | 6.65 | 6.65 | 49 |
| 11 | 7.9 | 5.6 | 43 |
| 7 | 5.575 | 7.93 | 40 |
| 14 | 9 | 4.92 | 34 |

2. Verfahren zur Herstellung der Verbindung nach Anspruch 1, umfassend:
    a) Inkontaktbringen von Rifapentin der Form I + SII mit Wasser oder einer Wasser enthaltenden Substanz oder Aussetzen einer relativen Luftfeuchtigkeit, die größer als 80 % ist, für mindestens 24 Stunden, oder alternativ
b) eine thermische Behandlung von Rifapentin der Form I + SII bei einer Temperatur zwischen 50° und 130°C.

3. Verfahren nach Anspruch 2, wobei der Kontakt mit Wasser durch Suspendieren von Rifapentin der Form I + SII in Wasser erhalten wird.

4. Verfahren nach Anspruch 3, wobei der Schritt (a) oder der alternative Schritt (b) unter vermindertem Druck durchgeführt werden.

5. Verfahren nach Anspruch 4, wobei der verminderte Druck im Bereich von 26.6 - 53.3 Pa liegt.

6. Verfahren nach Anspruch 2, 4 oder 5, wobei die thermische Behandlung bei einer Temperatur zwischen 70° und 80°C stattfindet.

7. Verfahren zur Herstellung der Verbindung nach Anspruch 1, umfassend:
a) Lösen irgendeiner Form von Rifapentin in einem niedrig siedenden Lösungsmittel unter Rühren bei Raumtemperatur, dann
b) Zugabe von Ethanol und teilweise Entfernen der flüchtigen Bestandteile durch Destillieren bei Umgebungsdruck bis eine gleichbleibende Temperatur des restlichen Gemisches von etwa 75° - 80°C erhalten wird,
c) Erhalten des so hergestellten Gemisches von Rifapentin der Form I + SII durch Filtration, und
d) Inkontaktbringen von Rifapentin der Form I + SII mit Wasser oder einer Wasser enthaltenden Substanz oder Aussetzen einer relativen Luftfeuchtigkeit, die größer als 80 % ist, für mindestens 24 Stunden unter vermindertem Druck, oder alternativ
e) eine thermische Behandlung von Rifapentin bei einer Temperatur zwischen 50° und 130°C unter vermindertem Druck.

8. Verfahren nach Anspruch 7, wobei der verminderte Druck im Bereich von 26.6 - 53.3 Pa liegt.

9. Pharmazeutische Zubereitung, die Rifapentin der reinen kristallinen Form I, wie in Anspruch 1 definiert, als Wirkstoff enthält.

10. Zubereitung nach Anspruch 9, wobei die pharmazeutische Zubereitung in einer oralen Dosierungsform vorliegt.

11. Zubereitung nach Anspruch 10, wobei die orale Dosierungsform eine Tablette oder eine Kapsel ist.

12. Verfahren zur Herstellung einer pharmazeutischen Zubereitung von Rifapentin, umfassend das Vermischen von Rifapentin der reinen kristallinen Form I, wie in Anspruch 1 definiert, mit einem pharmazeutisch verträglichen Träger.

13. Verfahren nach Anspruch 12, wobei die pharmazeutische Zubereitung in einer oralen Dosierungsform vorliegt.

14. Verfahren nach Anspruch 13, wobei die orale Dosierungsform eine Tablette oder eine Kapsel ist.

## Revendications

1. Forme cristalline pure I de la rifapentine, ayant les caractéristiques suivantes :
a) Caractérisation à l'état solide
La forme I apparaît à l'état de cristaux anisotropes (biréfringence), avec une constitution indéfinie.
b) L'analyse thermique (DSC-TG) est effectuée avec un appareil de thermoanalyse TA 2000 de Mettler ou 990 de DuPont et avec une thermobalance (figure 1), et les données relatives sont indiquées dans le tableau 1. Les conditions de travail sont les suivantes : débit de gaz $N_2$ = 25 ml/min, vitesse de balayage = 10°C/min, poids de l'échantillon = environ 4 mg.
La forme I présente un pic endothermique à 172°C (dû à la fusion), suivi directement d'une décomposition qui empêche de déterminer la chaleur de fusion d'une manière fiable ; un endotherme de désolvatation, qui est causé par l'eau d'imbibition, apparaît à 25-110°C.

c) On enregistre le spectre infrarouge (IR) avec un spectrophotomètre de Perkin Elmer Modèle 580 dans une pâte de Nujol, et les résultats sont indiqués sur la figure 2, tandis que la répartition des bandes fonctionnelles principales (cm$^{-1}$) est indiquée dans le TABLEAU II.

TABLEAU I

| Point de fusion ou température de transition (°C) | Perte de poids (%) |
|---|---|
| 25 - 110 * | 4 |
| 172 ** | - |

\* Eau d'imbibition.

\** Fusion suivie d'une décomposition rapide.

TABLEAU II

| Ansa ν OH | 3260*, 3440,3280 |
|---|---|
| Furanone ν C = O | 1730 |
| Acétyle ν C = O | 1705 |
| Amide ν C = O (amide I) | 1660, 1615 |
| Amide δ NH (amide II) | 1510 |
| Acétyle ν C-O-C | 1250 |

\* Bande étroite d'eau.

d) Le motif de diffraction des rayons X avec une poudre de la forme I d'après ASTM, obtenu avec un instrument Philips (PM 1010/77, 1049/01, 4025/10), rayonnement CuK$_{alpha}$, est indiqué dans le TABLEAU III et sur la figure 3.

## TABLEAU III

Longueurs d'onde :  ALPHA 1        = 1,54051 angström

ALPHA 2       = 1,54433

ALPHA - MOYENNE = 1,54178.

Les cinq réflexions les plus fortes sont :

| N° | Degré thêta | D Angström | Intensité |
|---|---|---|---|
| 1 | 2,95 | 14,98 | 100 |
| 9 | 6,65 | 6,65 | 49 |
| 11 | 7,9 | 5,6 | 43 |
| 7 | 5,575 | 7,93 | 40 |
| 14 | 9 | 4,92 | 34 |

2. Procédé de préparation du composé selon la revendication 1, comprenant :

a) une mise en contact de la rifapentine de forme I + SII avec de l'eau ou une substance contenant de l'eau ou une exposition à un taux d'humidité relative supérieur à 80 %, pendant au moins 24 heures, ou selon une autre possibilité:

b) un assujetissement de la rifapentine de forme I + SII à un traitement thermique à une température comprise entre 50 et 130°C.

3. Procédé selon la revendication 2, dans lequel on obtient le contact avec l'eau en mettant la rifapentine de forme I + SII en suspension dans de l'eau.

4. Procédé selon la revendication 3, dans lequel on effectue l'étape (a) ou l'étape alternative (b) à une pression réduite.

5. Procédé selon la revendication 4, dans lequel la pression réduite est comprise entre 26,6 et 53,3 Pa.

6. Procédé selon la revendication 2, 4 ou 5, dans lequel on effectue le traitement thermique à une température comprise entre 70 et 80°C.

7. Procédé de préparation du composé selon la revendication 1, comprenant :

a) une dissolution de toutes les formes de rifapentine dans un solvant à point bas d'ébullition, sous agitation, à la température ambiante, puis

b) une addition d'éthanol et une, évacuation partielle des substances volatiles, par distillation à la pression ambiante, jusqu'à ce qu'une température constante du mélange résiduel d'environ 75-80°C soit obtenue,

c) un recueil du mélange ainsi produit de rifapentine de forme I + SII, par filtration, et

d) une mise au contact de la rifapentine de forme I + SII avec de l'eau ou une substance contenant de l'eau ou une exposition à un taux d'humidité relative supérieur à 80 %, pendant au moins 24 heures, à une pression réduite, ou selon une autre possibilité :

e) un assujetissement de celle-ci à un traitement thermique à une température comprise entre 50 et 130°C, à une pression réduite.

8. Procédé selon la revendication 7 dans lequel la pression réduite est comprise entre 26,6 et 53,3 Pa.

9. Formulation pharmaceutique contenant de la rifapentine sous sa forme cristalline pure I telle que définie dans la revendication 1, comme un principe actif.

10. Formulation selon la revendication 9, dans laquelle la formulation pharmaceutique est sous une forme destinée à une administration orale.

11. Formulation selon la revendication 10, dans laquelle la forme pour une administration orale est un comprimé ou une capsule.

12. Procédé de préparation d'une formulation pharmaceutique de rifapentine comprenant le mélange de rifapentine de forme cristalline pure I telle que définie dans la revendication 1 avec un véhicule pharmaceutiquement acceptable.

13. Procédé selon la revendication 12, dans lequel la formulation pharmaceutique se présente sous une forme destinée à une administration orale.

14. Procédé selon la revendication 13, dans lequel la forme pour une administration orale est un comprimé ou une capsule.

FIG. 1

TG-DSC heating curves of rifapentine Form I

EP 0 536 197 B1

FIG. 2

IR spectrum of rifapentine Form I in nujol mull

20

FIG. 3    X-ray diffraction pattern of rifapentine Form I

FIG. 3    X-ray diffraction pattern of rifapentine Form I